# EUROPEAN PATENT SPECIFICATION

(11) **EP 4 349 273 B1**
(45) Date of publication and mention of the grant of the patent: **14.10.2026**
(21) Application number: 22894201.7
(22) Date of filing: 26.05.2022
(51) Int. Cl.: A61B 10/02

(54) **WINDOW OPENING SIZE ADJUSTMENT METHOD FOR SAMPLING WINDOW OF BIOPSY SURGICAL DEVICE AND CONTROL DEVICE**
FENSTERÖFFNUNGSGRÖSSENANPASSUNGSVERFAHREN FÜR EIN PROBENFENSTER EINER CHIRURGISCHEN BIOPSIEVORRICHTUNG UND STEUERUNGSVORRICHTUNG
PROCÉDÉ D'AJUSTEMENT DE TAILLE D'OUVERTURE DE FENÊTRE POUR UNE FENÊTRE D'ÉCHANTILLONNAGE D'UN DISPOSITIF CHIRURGICAL DE BIOPSIE, ET DISPOSITIF DE COMMANDE

(30) Priority: 16.11.2021 CN 202111355495
(43) Date of publication of application: 10.04.2024
(73) Proprietor: Chongqing Xishan Science & Technology Co., Ltd., Chongqing 401121 (CN)
(72) Inventor: GUO, Yijun, Chongqing 401121 (CN); LI, Mingxuan, Chongqing 401121 (CN); LI, Chaowei, Chongqing 401121 (CN); CAI, Li, Chongqing 401121 (CN)
(74) Representative: Mohun, Stephen John
(86) International application number: PCT/CN2022/095130
(87) International publication number: WO 2023/087657

(56) References cited:
- WO-A1-2016/090023
- CN-A- 104 188 693
- CN-A- 105 796 135
- CN-A- 105 796 135
- CN-A- 109 199 464
- CN-A- 112 587 098
- CN-A- 114 027 888
- CN-U- 213 606 537
- CN-U- 213 606 537
- CN-U- 214 157 384
- CN-U- 214 157 384
- CN-U- 216 495 408
- US-A1- 2016 228 105
- US-A1- 2019 365 361
- US-A1- 2020 015 794
- US-A1- 2020 187 921

## Description

### TECHNICAL FIELD

The present application relates to the technical field of medical devices, and particularly to a method and control device for adjusting an opening size of a sampling window of a biopsy surgical device.

### BACKGROUND

Biopsy is a technique that removes diseased tissue from the patient's body for pathological examination by cutting, clamping, or puncturing for diagnosis and treatment needs. Biopsy surgical devices are used to sample biological tissue from the human body. A widely used biopsy surgical device is the one that performs sampling through rotational cutting, which typically includes a cutter and a handle. The cutter includes an inner cutter tube and an outer cutter tube that are sleeved. The front end of the outer cutter tube is a tip for puncture. A sampling slot is defined on the side of the outer cutter tube near the front end. The front end of the inner cutter tube includes a cutting edge. During puncture, the inner cutter tube is located at the forefront and closes the sampling slot. When the puncture is in position, the inner cutter tube moves backward to expose the sampling slot. The tissue is sucked into the sampling slot under negative pressure. At this time, the inner cutter tube moves forward and performs rotational cutting, thereby cutting off the tissue entering the sampling slot and accommodating it into the front end of the inner cutter tube.

CN214157384U provides a biopsy surgical device including a cutter assembly and a handle. The cutter assembly has an outer cutter tube and an inner cutter tube nested inside the outer cutter tube, with a sampling slot provided on the side wall of the front end of the outer cutter tube. The handle consists of a housing, a cutter driving device, and a handle communication module. The cutter driving device can drive the inner cutter tube to rotate and move axially, and can adjust the opening size of the sampling slot by controlling the axial movement of the inner cutter tube according to the control signal from the host. The handle is also equipped with an opening adjustment device, which includes a user operation unit and a handle controller. The user operation unit is arranged on the front face, side face, or top face of the housing and connected to the input end of the handle controller. The output end of the handle controller is connected to the handle communication module, which can obtain the opening adjustment signal from the user operation unit, convert it into a digital signal, and then send the digital signal to the host via the handle communication module, and the host sends a control signal to the cutter driving device. The user operation unit is a key module, including a first key (for increasing the opening size of the sampling slot) and a second key (for decreasing the opening size of the sampling slot). The handle controller is provided with an electrical detection interface connected to the output end of the key module; short-pressing the keys adjusts a set size (e.g., 1mm), while long-pressing enables continuous adjustment. The handle is provided with a display module connected to the handle controller, and the display module includes a digital display screen arranged on the top face of the housing, which can display the value or percentage of the opening size of the sampling slot. The handle communication module includes a cable and a connector assembly; one end of the cable is connected to the cutter driving device and the handle controller, and the other end is connected to the connector assembly, and a wireless communication module can also be used instead. The corresponding biopsy surgical system includes the aforementioned biopsy surgical device and a host. The host includes a host controller and a host communication module, where the host controller is connected to the host communication module, and the host communication module is in communication connection with the handle communication module, capable of receiving digital signals, generating control signals, and sending them to the cutter driving device.

US2020/187921A1 provides a single insertion multiple sample biopsy apparatus including a driver assembly and a biopsy probe assembly, where the biopsy probe assembly is releasably attached to the driver assembly. The driver assembly has an electromechanical power source, a vacuum source, and a controller circuit. The electromechanical power source includes a cutter module, a transport module, and a piercing module; the cutter module includes a motor (with a drive gear), the transport module includes a motor (with a drive gear), and the piercing module includes a motor, a drive spindle, and a piercing shot driver. The controller circuit includes a processor circuit and a memory circuit, and is electrically and communicatively connected to the electromechanical power source, the vacuum source, a vacuum sensor, and a user interface; a virtual energy reservoir can be established in the memory circuit. The biopsy probe assembly includes a vacuum cannula, a stylet cannula, and a cutter cannula arranged coaxially; the vacuum cannula is located inside the stylet cannula, and the stylet cannula is located inside the cutter cannula. The vacuum cannula is in fluid communication with the vacuum source and has an elongate portion and a flared portion (including a first flared section and a second flared section) extending distally from the elongate portion. The stylet cannula is drivingly connected to the electromechanical power source and is movable relative to the vacuum cannula between a first extended position and a first retracted position; a sample notch and a proximally extending protrusion member are provided on its distal portion, and the protrusion member is received in the flared portion of the vacuum cannula when retracted. The cutter cannula is drivingly connected to the electromechanical power source and is movable relative to the stylet cannula between a second extended position (covering the sample notch) and a second retracted position (exposing the sample notch). The stylet cannula is drivingly connected to the transport module through a stylet gear-spindle set, and the cutter cannula is drivingly connected to the cutter module through a cutter gear-spindle set; the pitch of the threaded spindle of the gear-spindle set determines the number of motor revolutions corresponding to the axial movement distance. The controller circuit executes program instructions, which can determine the axial movement distance of the cannula by calculating the number of motor shaft revolutions and control the motor rotation to drive the cannula movement; it can also monitor the motor current, control the current based on the state of the virtual energy reservoir, determine whether it enters dense tissue, and repeatedly move the protrusion member of the stylet cannula into and out of the flared portion of the vacuum cannula to assist in tissue sample transport, and the vacuum source continuously applies vacuum through the vacuum cannula.

CN 213606537 U provides a biopsy surgical device including a cutter assembly and a handle. The cutter assembly has an outer cutter tube and an inner cutter tube nested inside the outer cutter tube, with a sampling slot provided on the side wall of the front end of the outer cutter tube. The handle consists of a housing, a cutter driving device, and a handle communication module. The cutter driving device can drive the inner cutter tube to rotate and move axially, and can adjust the opening size of the sampling slot by controlling the axial movement of the inner cutter tube according to the control signal from the host. The handle is also equipped with an opening adjustment device, which includes an adjustable potentiometer and a handle controller. The adjustable potentiometer is connected to the input end of the handle controller, and the output end of the handle controller is connected to the handle communication module. The handle controller can obtain the state signal of the adjustable potentiometer, convert it into a digital signal, and then send the digital signal to the host via the handle communication module, and the host sends a control signal to the cutter driving device. The adjustable potentiometer is a mechanical potentiometer, and the handle controller is provided with an A/D port connected to the output end of the adjustable potentiometer; the adjustable potentiometer can be of knob type, toggle type, or sliding type. The housing is provided with an opening, the adjustable potentiometer is located inside the housing, and its operating part is exposed from the opening; the opening can be arranged on the front face, side face, or top face of the housing. The handle is provided with a display module connected to the handle controller, and the display module includes a digital display screen. The handle controller converts the digital signal into the value of the opening size of the sampling slot and sends it to the digital display screen for display. The digital display screen and the adjustable potentiometer are arranged axially and close to the adjustable potentiometer. The handle communication module includes a cable and a connector assembly; one end of the cable is connected to the control signal input end of the cutter driving device and the handle controller, and the other end is connected to the connector assembly, and a wireless communication module can also be used instead. The corresponding biopsy surgical system includes the aforementioned biopsy surgical device and a host. The host includes a host controller and a host communication module, where the host controller is connected to the host communication module, and the host communication module is connected to the handle communication module, capable of receiving digital signals, generating control signals based on the digital signals, and then sending them to the cutter driving device via the host communication module and the handle communication module.

During the surgery, it is necessary to accurately control the opening size of the sampling slot according to the amount of sampling.

### SUMMARY

The present invention is defined in the attached independent claims, to which reference should now be made.

### BRIEF DESCRIPTION OF THE DRAWINGS

In order to illustrate the technical solutions of the embodiments of the present application or prior art more clear, the accompanying drawings used in the description of the embodiments or prior art will be briefly introduced below. It is apparent that the accompanying drawings described herein are only some embodiments of the present application. For those of ordinary skill in the art, other drawings can also be obtained from these drawings without creative efforts.
FIG. 1 is a flow chart according to an embodiment.
FIG. 2 is a schematic structural diagram of a biopsy surgical device according to an embodiment.
FIG. 3 is a schematic structural diagram of an outer cutter tube and an inner cutter tube according to an embodiment.
FIG. 4 is a schematic structural diagram showing an inner cutter tube exposing/covering part of a sampling window according to an embodiment.
FIG. 5 is a schematic structural diagram showing a sampling window that is completely opened according to an embodiment.
FIG. 6 is a schematic diagram showing a relationship between an opening length of a sampling window and an axial movement distance of an inner cutter tube according to an embodiment.
FIG. 7 is a schematic diagram showing a relationship between an opening length of a sampling window and an axial movement distance of an inner cutter tube according to another embodiment.
FIG. 8 is a schematic diagram showing a transmission of an inner cutter tube according to an embodiment.
FIG. 9 is a schematic diagram showing a transmission of an inner cutter tube according to another embodiment.
FIG. 10 is a schematic diagram showing a transmission of an inner cutter tube according to yet another embodiment.
FIG. 11 is a schematic diagram showing a transmission of an inner cutter tube according to yet another embodiment.
FIG. 12 is a partially enlarged view of FIG. 11.
FIG. 13 is an axial view showing the mating of a sleeve piece with an extension section according to an embodiment.

### DETAILED DESCRIPTION OF THE EMBODIMENTS

The implementation of the present application is described below in conjunction with specific embodiments. Those familiar with this technology can easily know other advantages and effects of the present application from the content disclosed in the present specification.

### Embodiments

In the present application, the orientation "front" and "back" are defined with reference to the usage status of the biopsy surgical device. During usage, the side facing the patient is the front, and the side away from the patient is the back.

During a biopsy surgery, the retraction position and distance of the inner cutter tube are controlled according to the amount of sampling so as to adjust the opening size of the sampling window. As known to the applicant, there are typically only 3 to 4 fixed adjustment positions, and adjustments can only be made between these fixed positions. For example, the sampling window can be opened at 50%, 100%, etc. It is impossible to achieve a refined and continuous adjustment of the opening size of the window. It only adapts to several fixed sampling lengths, resulting in poor adaptability and weak universality of the biopsy surgical device.

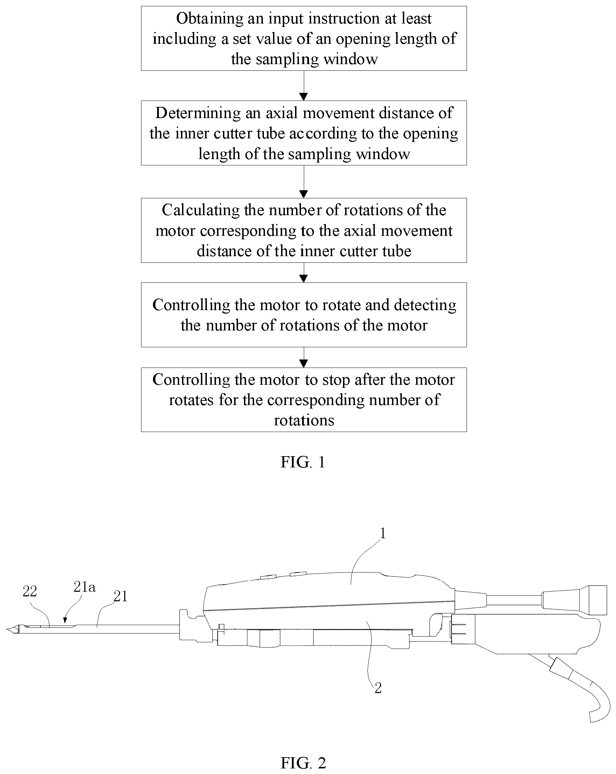

As shown in FIGS. 1 to 6, the present embodiment illustrates a biopsy surgical device, including a handle 1 and a cutter assembly 2 that are connected. The cutter assembly 2 includes an outer cutter tube 21 and an inner cutter tube 22 that are arranged coaxially. The front end of the outer cutter tube 21 includes a puncture tip. A sampling window 21a is defined on the side of the outer cutter tube 21 near the front end. The front end of the inner cutter tube 22 includes a cutting edge. The inner cutter tube 22 and the outer cutter tube 21 are sleeved. The inner cutter tube 22 is inserted in the outer cutter tube 21, or the inner cutter tube 22 is sleeved over the outer cutter tube 21. The inner cutter tube 22 can move axially relative to the outer cutter tube 21.

A motor 11 is arranged in the handle 1. The motor 11 is connected to the inner cutter tube 22 through a transmission mechanism so as to drive the inner cutter tube 22 to move along the axial direction (front and back direction) and control the front end of the inner cutter tube to stop at any position along the axial direction of the sampling window of the outer cutter tube, such that the length of the sampling window 21a that is exposed or covered by the inner cutter tube can be continuously adjusted, thereby adjusting the actual opening length of the sampling window 21a for tissue aspiration, i.e., the axial dimension of the actual used sampling window 21a.

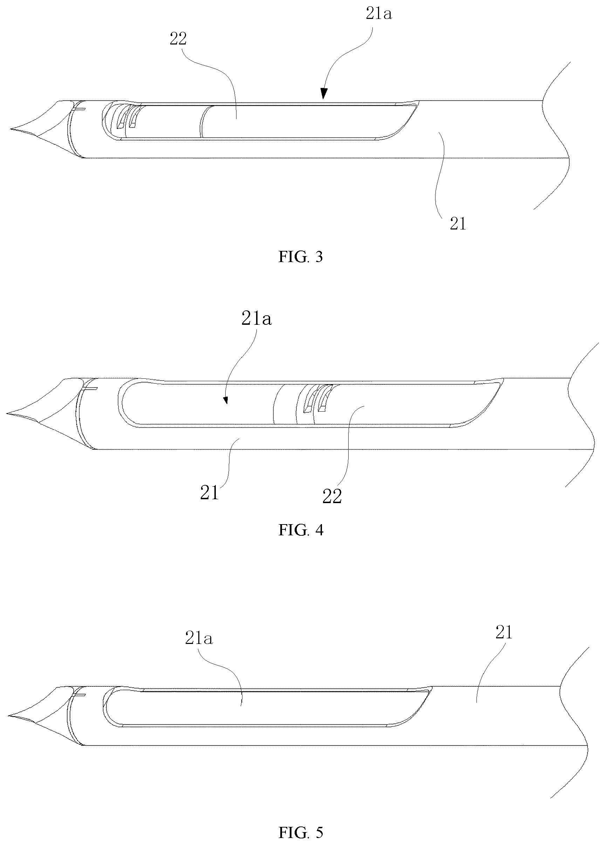

During puncture, the inner cutter tube 22 is located at the forefront and closes the sampling window 21a. When the puncture is in position, the inner cutter tube 22 moves backward to expose the sampling window 21a. The tissue is sucked into the sampling window 21a under negative pressure. At this time, the inner cutter tube moves forward or rotates while moving forward, so as to cut off the tissue entering the sampling window 21a and then transport the cut tissue to a sample collection box through the inner cutter tube under atmospheric pressure. The position of the inner cutter tube 22 can be adjusted between completely covering the sampling window 21a (the state shown in FIG. 4) and completely exposing the sampling window 21a (the state shown in FIG. 5), so that the actual opening size of the sampling window 21a can be continuously adjusted according to the sampling requirements.

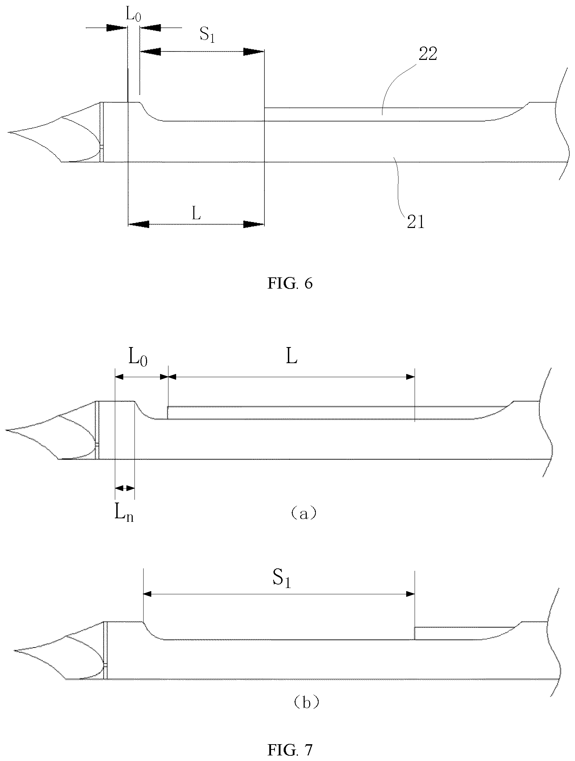

A method for adjusting the opening size of a sampling window of a biopsy surgical device includes obtaining an input instruction. The input instruction at least includes a set value of an opening length of the sampling window. For example, a window opening length parameter can be input through buttons or a touch screen on the handle 1. Alternatively, a relative amount of increase/decrease, i.e., an adjustment value of the window opening length, is input. The set value of the opening length of the sampling window can be calculated according to the input adjustment value. Then, an axial movement distance of the inner cutter tube 22 is determined according to the actual required window opening length, i.e., according to the current position of the inner cutter tube 22 and the set value of the opening length of the sampling window. For example, when the front end of the inner cutter tube 22 is flush with the front end of the sampling window 21a, the distance the inner cutter tube 22 moves backward is the window opening length. When the front end of the inner cutter tube 22 is forward beyond the front end of the sampling window 21a, the distance the inner cutter tube 22 moves backward needs to be greater than the input window opening length, and the difference is the distance between the front end of the inner cutter tube 22 and the front end of the sampling window 21a. This distance (i.e., the difference) is determined when the biopsy surgical device is manufactured. The movement distance of the inner cutter tube 22 is determined according to the relative position, as shown in FIG. 6 and FIG. 7.

Then, the number of rotations of the motor corresponding to the axial movement distance of the inner cutter tube 22 is calculated according to a transmission relationship of the transmission mechanism, such that the motor is controlled to rotate according to the number of rotations, casing the inner cutter tube 22 moves axially until the sampling window 21a reaches the specified window opening length.

The number of rotations of the motor can be detected by a Hall sensor, and the motor stops rotating after completing the specified number of rotations.

An input device, a driver, and a Hall sensor are connected to the controller, respectively. The input device may be a button, a touch screen, etc. The controller stores a calculation method for the relationship between the axial movement distance of the inner cutter tube 22 and the number of rotations of the motor. When obtaining the input instruction from the input device, the controller calculates the distance that the inner cutter tube 22 needs to move along the axial direction according to the relative position relationship between the inner cutter tube 22 and the sampling window 21a. Then, the number of rotations that the motor needs to rotate is determined according to the transmission relationship of the current transmission mechanism. The driver drives the motor to rotate according to the determined number of rotations. At the same time, the Hall sensor detects the number of rotations of the motor and feeds it back to the controller. The number of rotations of the motor is obtained in real time. When the corresponding number of rotations is reached, the controller controls the motor to stop through the driver. The position of the inner cutter tube 22 can be adjusted between completely covering the sampling window 21a and completely exposing the sampling window, so that the actual opening length of the sampling window 21a can be adjusted to adapt to the needs of different sampling sizes. In addition, based on the relationship between the axial movement distance of the inner cutter tube 22 and the number of rotations of the motor, the opening length of the sampling window 21a can be accurately controlled and can be adjusted in real time as needed by controlling the number of motor rotations.

The controller may be a general-purpose processor, including a Central Processing Unit (CPU), a Network Processor (NP), etc. The controller may also be a Digital Signal Processor (DSP), an Application-Specific Integrated Circuit (ASIC), a Field-Programmable Gate Array (FPGA) or other programmable logic devices, a discrete gate or transistor logic device, or a discrete hardware component.

As shown in FIG. 6, when the current position of the inner cutter tube 22 is the initial position (i.e., the inner cutter tube is located at the forefront and closes the sampling window), the axial movement distance L of the inner cutter tube 22 is determined as *L = S*₁ + *L*₀, where *S*₁ is the input set value of opening length of the sampling window, and *L*₀ is the distance from the front end of the inner cutter tube 22 to the front end of the sampling window 21a when the inner cutter tube 22 is at the initial position.

When the current position of the inner cutter tube 22 is not at the initial position, there are two cases. When *Lₙ* ≤ *L*₀*,* it means that the inner cutter tube 22 is located between the initial position and the front end of the sampling window 21a. The axial movement distance L of the inner cutter tube 22 is determined as *L* = *S*₁ *+* (*L*₀ *- Lₙ*), and the distance to be moved is greater than the set value. When *Lₙ* ≥ *L*₀*,* it means that the inner cutter tube 22 is located behind the front end of the sampling window 21a, the distance to be moved is less than the set value, and *L = S*₁ *-* (*Lₙ - L*₀)*. Lₙ* is the distance from the front end of the inner cutter tube 22 to the initial position. The inner cutter tube 22 moves backward when L>0, and the inner cutter tube 22 moves forward when L<0.

As shown in FIG.7, (a) shows the current position of the inner cutter tube 22, and (b) shows the final adjustment position of the inner cutter tube 22. The current position of the inner cutter tube 22 is behind the front end of the sampling window 21a, and the axial movement distance of the inner cutter tube 22 is *L* = *S*₁ *-* (*Lₙ - L*₀)*.* The calculation method is similar when *Lₙ* ≤ *L*₀*.*

Regarding the transmission structure of the biopsy surgical device, there are different transmission ratio calculation methods according to different transmission structures.

In an embodiment, the output end of the motor 11 and the inner cutter tube 22 are directly connected through threads, and the inner cutter tube 22 moves axially. When the motor 11 completes one revolution, the inner cutter tube 22 moves axially by one pitch P. The number X of rotations of the motor corresponding to the axial movement distance L of the inner cutter tube 22 is obtained by $X = \frac{L}{P}$*,* where P is the pitch of the thread of the inner cutter tube 22 that matches the output end of the motor 11. When X>0, the motor rotates forward and the inner cutter tube 22 moves backward. When X<0, the motor rotates in reverse and the inner cutter tube 22 moves forward. X is not necessarily an integer, in other words, the motor can rotate half a turn or a quarter of a turn. The number of rotations of the motor can be set according to actual needs.

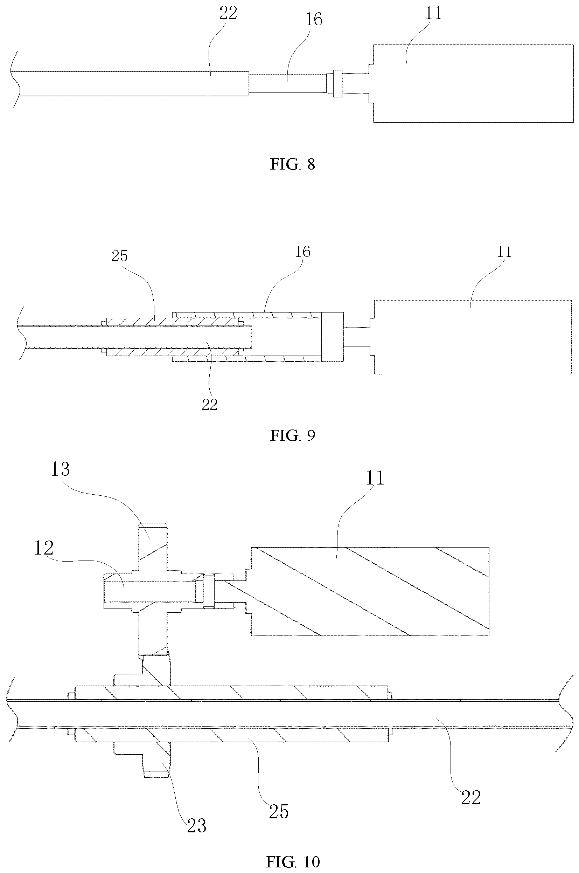

Specifically, as shown in FIG. 8, the output end of the motor 11 is fixedly connected to a transmission member 16 that rotates synchronously with it. The transmission member 16 includes a first thread segment. The first thread segment may be an inner screw thread or an outer screw thread. The back portion of the inner cutter tube 22 includes a second thread segment that matches the first thread segment, and P is the pitch of the first thread segment or the second thread segment. The transmission member 16 is directly threaded with the back portion of the inner cutter tube 22. A limiting element is set on the cutter assembly or the handle to limit the rotation of the inner cutter tube 22 so that the inner cutter tube 22 moves axially.

As shown in FIG. 9, in another embodiment, the output end of the motor 11 is fixedly connected to a transmission member 16 that rotates synchronously with it. The transmission member 16 includes a first thread segment. The first thread segment may be an inner screw thread or an outer screw thread. A transmission sleeve 25 is arranged on the inner cutter tube 22. The transmission sleeve 25 includes a second thread segment that matches the first thread segment. The transmission member 16 and the transmission sleeve 25 are in a threaded connection. The transmission sleeve 25 and the inner cutter tube 22 are arranged to be axially fixed and circumferentially rotate relative to each other, so that when the motor 11 rotates, the inner cutter tube 22 is driven to move axially through the transmission sleeve 25, and the front end of the inner cutter tube 22 can stop at any position along the axial direction of the sampling window 21a of the outer cutter tube 21. This embodiment is applicable for the biopsy surgical device in which the inner cutter tube 22 does not rotate but only moves axially. A limiting element may be set on the cutter assembly or the handle to limit the rotation of the inner cutter tube 22.

As shown in FIG. 10, in an embodiment, the transmission mechanism includes a first driving gear 13 and a first driven gear 23 that mesh with each other. The output end of the motor 11 is connected with an output shaft 12. The first driving gear 13 is sleeved on the output shaft 12 and rotates with the output shaft 12. The inner cutter tube 22 includes an outer thread segment (i.e., a third thread segment). The inner wall of the first driven gear 23 includes inner threads (i.e., a fourth thread segment). The first driven gear 23 is sleeved on the outer thread segment of the inner cutter tube 22 and is threadedly engaged with the inner cutter tube 22. By the cooperation of the threaded pair, the inner cutter tube 22 is driven to move axially. The first motor 11 drives the first driving gear 13 to rotate, causing the first driven gear 23 to drive the inner cutter tube 22 to move axially and stop the front end of the inner cutter tube 22 at any position in the axial direction of the sampling window 21a of the outer cutter tube 21, thereby changing the size of the sampling window 21a exposed or covered by the inner cutter tube 21.

In this embodiment, to facilitate manufacturing, the transmission sleeve 25 is fixedly sleeved on the inner cutter tube 22. The transmission sleeve 25 is provided with outer threads to cooperate with the first driven gear 23. In other embodiments, a shaft portion with outer threads extends axially from the first driven gear 23. The shaft portion inserts into the inner cutter tube 22 and is threadedly engaged therewith.

The above embodiment is applicable for the biopsy surgical device in which the inner cutter tube 22 rotates while cutting, and for the biopsy surgical device in which the inner cutter tube 22 does not rotate but only moves axially.

In the above structure, by the transmission of the gear set and the threads, after the first driven gear 23 rotates one turn, the inner cutter tube 22 moves axially by one pitch, and the motor 11 rotates D/B turns, then the number X of rotations of the motor 11 corresponding to the axial movement distance L of the inner cutter tube 22 is obtained by $X = \frac{L}{{P}_{1} \frac{B}{D}}$*,* where *P*₁ is the pitch of the third thread segment or the fourth thread segment, B/D is the transmission ratio between the first driving gear 13 and the first driven gear 23, for example, B is the number of teeth of the first driving gear, and D is the number of teeth of the first driven gear.

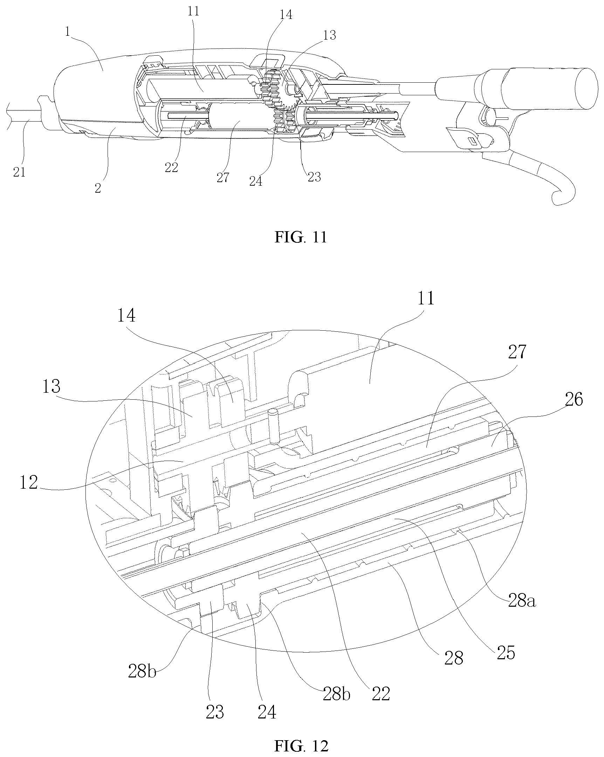

As shown in FIG. 11, in an embodiment, the transmission mechanism includes a first transmission structure and a second transmission structure. The motor drives the inner cutter tube 22 to rotate around the axis thereof through the second transmission structure. An output portion of the first transmission structure is sleeved on the inner cutter tube 22 and is threadedly engaged with the inner cutter tube 22. The output portion and the inner cutter tube 22 rotate in the same direction, and there is a speed difference between the output portion and the inner cutter tube 22. The inner cutter tube 22 is driven to move axially by the speed difference and the threaded structure. Therefore, a slower movement adjustment in the axial direction is achieved while the inner cutter tube 22 rotates at a high speed, which facilitates precise adjustment.

As shown in FIG. 11 and FIG. 12, the first transmission structure includes a first driving gear 13 and a first driven gear 23 that mesh with each other. The first driven gear 23 is the output portion of the first transmission structure. The output end of the motor 11 is connected with an output shaft 12. The first driving gear 13 is sleeved on the output shaft 12 and rotates with the output shaft 12. The inner cutter tube 22 includes an outer thread segment (a fifth thread segment), and the inner wall of the first driven gear 23 includes inner threads (a sixth thread segment). The first driven gear 23 is sleeved on the outer thread segment of the inner cutter tube 22 and is threadedly engaged with the inner cutter tube 22.

Torque is transmitted between the motor 11 and the inner cutter tube 22 through the second transmission structure to drive the inner cutter tube 22 to rotate. The second transmission structure includes a second driving gear 14 and a second driven gear 24 that mesh with each other. The second driving gear 14 is arranged on the output shaft 12 of the motor 11 and rotates synchronously with the output shaft 12. The second driven gear 24 is coaxially sleeved on the inner cutter tube 22 and is arranged to be circumferentially fixed and axially slidable relative to the inner cutter tube 22. In other words, the inner cutter tube 22 rotates with the second driven gear 24, but the inner cutter tube 22 can slide axially relative to the second driven gear 24 and still transmit torque during sliding.

Specifically, the transmission mechanism also includes a transmission sleeve 25 fixedly sleeved on the inner cutter tube 22. The transmission sleeve 25 includes outer threads. The first driven gear 23 is sleeved on the transmission sleeve 25 and is threadedly engaged with the transmission sleeve 25.

In the above structure, the inner cutter tube 22 is driven by the transmission mechanism to rotate around the axis thereof, i.e., rotate synchronously with the second driven gear 24. At the same time, the first driven gear 23 is threadedly engaged with the inner cutter tube 22, and the inner cutter tube 22 and the first driven gear rotate in the same direction but at different speeds. There is a speed difference, which drives the inner cutter tube 22 to move axially through the threads.

In an embodiment, an extension segment 26 extends axially from the transmission sleeve 25. One end of the second driven gear 24 is fixedly connected with a sleeve piece 27. The sleeve piece 27 is sleeved on the extension segment 26 and cooperates with the extension section 26 through a convex-concave structure to transmit torque. In addition, the transmission sleeve 25 can slide axially relative to the sleeve piece 27 to maintain torque transmission during the relative axial movement.

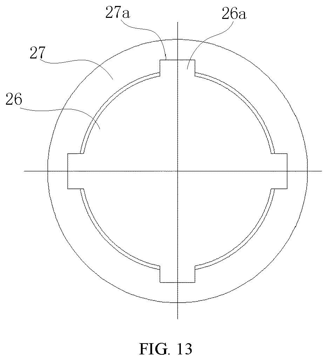

As shown in FIG. 13, in an embodiment, a groove 27a is defined in the inner wall of the sleeve piece 27 along the axial direction of the inner cutter tube 22. Specifically, a plurality of grooves 27a are distributed along the circumferential direction. Protrusions 26a corresponding to the grooves 27a are provided on the extension segment 26, and the length of the groove 27a along the axial direction of the inner cutter tube 22 is greater than or less than the length of the protrusion 26a along the axial direction of the inner cutter tube 22. Alternatively, the extension segment 26 and the sleeve piece 27 may also be matched by keyway or spline.

In this embodiment, the cutter assembly 2 also includes a support housing 28. The inner cutter tube 22 and the outer cutter tube 21 are both mounted on the support housing 28. The support housing 28 includes a transmission window. The first driven gear 23 and the second driven gear 24 partially extend out of the support housing 28 through the transmission window. The support housing 28 includes a positioning structure configured to axially position the first driven gear 23 and the second driven gear 24, so that the first driven gear 23 and the second driven gear 24 are fixed in the axial direction and only perform rotational movement. In this embodiment, the axial positioning is achieved by the steps 28b formed by the inner wall of the support housing 28 and the transmission window. In order to reduce friction while ensuring support, a plurality of convex ribs 28a are provided on the inner wall of the support housing 28, and the outer wall of the sleeve piece 27 is supported by the convex ribs 28a.

Based on the above transmission structure, the process of calculating the number of rotations of the motor according to the axial movement distance L of the inner cutter tube 22 is as follows.

The first driving gear 13 and the second driving gear 14 are both connected to the output shaft of the motor and rotate at the same speed. The number of teeth on the first driving gear 13, the second driving gear 14, the first driven gear 23, and the second driven gear 24 are different, resulting in different transmission ratios for the two gear sets. Therefore, the speeds of the first driven gear 23 and the second driven gear 24 are different, and the speed of the second driven gear 24 is the same as that of the inner cutter tube 22 rotating around the axis thereof.

The speed difference between the first driven gear 23 and the second driven gear 24 is *N*₁: ${N}_{1} = \frac{B}{D} N - \frac{A}{C} N = \left(\frac{B}{D}-\frac{A}{C}\right) N$ where N is the speed of the first driving gear 13 and the second driving gear 14, $\left(\frac{B}{D}-\frac{A}{C}\right)$ is the speed difference coefficient. B/D is the transmission ratio between the first driving gear 13 and the first driven gear 23, which may be a ratio of the number of teeth of the gears, or the reciprocal of the radius ratio. A/C is the transmission ratio between the second driving gear 14 and the second driven gear 24, which may be the ratio of the number of teeth of the gears, or the reciprocal of the radius ratio.

From the above formula, it can be seen that the first driven gear 23 rotates faster than the second driven gear 24 by *N*₁. The transmission sleeve 25 and the second driven gear 24 rotate synchronously and have the same rotation speed, i.e., the first driven gear 23 rotates faster than the transmission sleeve 25 by *N*₁. Since the second driven gear 24 and the first driven gear 23 are axially fixed, the speed difference between the first driven gear 23 and the transmission sleeve 25 causes the transmission sleeve 25 to move forward or backward through the thread of the first driven gear 23 (the forward or backward movement is achieved by changing the rotation direction of the motor). Due to the axial fixation between the inner cutter tube 22 and the transmission sleeve 25, the forward or backward movement of the inner cutter tube 22 is achieved.

The speed of the first driven gear 23 may be higher than or lower than the speed of the second driven gear 24. The speed of the driven gears and the rotation direction of the motor jointly determine whether the inner cutter tube 22 moves forward or backward.

The output speed *N*₂ of the inner cutter tube 22 is as follows: ${N}_{2} = \frac{A}{C} N$*,* which is the same as the speed of the second driven gear 24.

The speed of forward and backward movement of the inner cutter tube 22 is related to the speed difference between the two driven gears and the pitch of the thread. The feed rate V of the inner cutter tube 22 is as follows: $V = \frac{{N}_{1} \times {P}_{2}}{60}$*,* where *P*₂ is the pitch of the outer thread of the transmission sleeve 25 or the pitch of the inner thread of the first driven gear 23. The transmission sleeve 25 has the same pitch as the first driven gear 23.

When the first driven gear 23 rotates one turn relative to the second driven gear 24, the inner cutter tube 22 moves forward by one pitch P. Since the speed difference coefficient between the first driven gear 23 and the second driven gear 24 is $\left(\frac{B}{D}-\frac{A}{C}\right)$, the second driving gear 14, the first driving gear 13, and the motor all rotate Y turns, where $Y = 1 / \left(\frac{B}{D}-\frac{A}{C}\right)$*.* The first driven gear 23 and the second driven gear 24 are one turn apart, and the inner cutter tube 22 moves forward by pitch *P*₂.

When the axial movement distance of the inner cutter tube 22 is L, the second driving gear 14, the first driving gear 13, and the motor all rotate X circles, then $\frac{{P}_{2}}{Y} = \frac{L}{X}$ and $X = \frac{L}{{P}_{2} \left(\frac{B}{D}-\frac{A}{C}\right)}$*.* The first driven gear 23 rotates $\frac{B}{D} X$ turns, and the second driven gear 24 rotates $\frac{A}{C} X$ turns. Therefore, in the case that the axial movement distance of the inner cutter tube 22 is known, the corresponding number of rotations of the motor can be calculated. By counting with a Hall sensor, the motor stops rotating after the specified number of rotations is reached. When there are multiple stages of gear transmission, the transmission ratio can be calculated in a similar way.

To facilitate the doctor's intuitive operation, the minimum unit for adjusting the opening length of the sampling window 21a along the axial direction is E, i.e., the inner cutter tube 22 can be moved along the axial direction by one minimum unit E at a time. The value of the minimum unit E ranges from 0.1mm to 2mm, such as 0.1mm, 1mm, 1.5mm, 2mm, etc. It is possible to move several minimum units in succession. For example, when the minimum adjustment unit of the window opening length of a biopsy surgical device is 0.1mm, each movement can increase or decrease by 0.1mm, and several 0.1mm can be continuously increased or decreased. The minimum unit of each biopsy surgical device has a unique value. The distance of each movement is the same, achieving a continuous and fine adjustment of the movement distance, so as to achieve a continuous adjustment of the actual opening length of the sampling window 21a.

During the adjustment process, the final parameter of the window opening length, such as 25mm, may be directly input. Alternatively, incremental parameters may be input, such as increasing or decreasing 5mm on the basis of the original window opening length.

During the operation of the biopsy surgical device, before and during puncture, the inner cutter tube 22 moves to the frontmost position to close the sampling window 21a. After the puncture is in place, the motor 11 drives the inner cutter tube 22 to move backward according to the sampling size requirement until the actual opening length of the sampling window 21a corresponds to the input value. Then, the tissue is sucked into the sampling window 21a from the back end of the inner cutter tube 22 through a negative pressure device. After this, the inner cutter tube 22 is driven to move forward and rotate at a high speed to cut off the tissue and accommodate it at the front end of the inner cutter tube 22, and the sampling is completed.

In this embodiment, the position of the inner cutter tube 22 can be continuously adjusted between completely covering the sampling window 21a and completely exposing the sampling window 21a, thereby adjusting the actual opening length of the sampling window 21a, and achieving continuous forward and backward adjustment of the inner cutter tube 22. Therefore, the opening length of the sampling window 21a can be adjusted in real time and continuously according to the sampling requirements, so as to meet precise resection needs of different sizes of lesions. In specific embodiments, the minimum opening can be only 5 mm, which meets the needs for precise resection of tiny lesions and preserves surrounding normal tissues to the greatest extent. The maximum opening can reach 30 mm, allowing for a larger sample size to be obtained in a single resection, improving resection efficiency, reducing surgical time, and meeting the surgical needs of larger lesions.

In an embodiment, a control device for adjusting an opening size of a sampling window of a biopsy surgical device includes an obtaining module, a distance calculation module, a rotation number calculation module, and a control module. The obtaining module is configured to obtain input instructions, which at least include a setting value of a sampling window opening length. The distance calculation module is configured to determine an axial movement distance of the inner cutter tube according to the current position of the inner cutter tube and the set value of the opening length. The rotation number calculation module is configured to calculate the number of rotations of the motor corresponding to the axial movement distance of the inner cutter tube according to a transmission relationship of the transmission mechanism. The control module is configured to control the motor to rotate according to the number of rotations, causing the inner cutter tube to move axially and achieving the target opening length of the sampling window.

Specific features of the control device for adjusting an opening size of a sampling window of a biopsy surgical device can be referred to the features of the method for adjusting an opening size of a sampling window of a biopsy surgical device, which will not be repeated here. The modules in the above-mentioned control device for adjusting an opening size of a sampling window of a biopsy surgical device may be implemented in whole or in part by software, hardware, and combinations thereof. Each of the above modules may be embedded in or independent of a processor of a computer device in a form of hardware, or may be stored in a memory of the computer device in a form of software, so as to be called by the processor to perform the operations corresponding to the above modules. It should be noted that the division of modules in the embodiments of the present application is schematic and is only a logical function division. In actual implementation, there may be other division manners.

In an embodiment, a computer-readable storage medium is provided, in which a computer program is stored. The computer program, when executed by a processor, the steps of the methods in the above embodiments are implemented.

## Claims

1. A method for adjusting an opening size of a sampling window of a biopsy surgical device,
the biopsy surgical device comprising a motor (11), an outer cutter tube (21), an inner cutter tube (22), and a controller, the outer cutter tube (21) being sleeved over the inner cutter tube (22), the sampling window (21a) being defined on a side of a front end of the outer cutter tube (21), the motor (11) being configured to drive the inner cutter tube (22) to move axially through a transmission mechanism and control a front end of the inner cutter tube (22) to stop at any position along an axial direction of the sampling window (21a) of the outer cutter tube (21), thereby adjusting an opening length of the sampling window (21a);
wherein the method is applied to the controller, **characterized in that** the method comprises:
obtaining an input instruction which at least comprises a set value of an opening length of the sampling window (21a);
determining an axial movement distance of the inner cutter tube (22) according to a current position of the inner cutter tube (22) and the set value of the opening length of the sampling window (21a);
calculating a number of rotations of the motor (11) corresponding to the axial movement distance of the inner cutter tube (22) according to a transmission relationship of the transmission mechanism; and
controlling the motor (11) to rotate according to the number of rotations, causing the inner cutter tube (22) to move axially, so that the sampling window (21a) reaches a target window opening length;
when the current position of the inner cutter tube (22) is an initial position where the front end of the inner cutter tube (22) closes the sampling window (21a), the axial movement distance of the inner cutter tube (22) is determined as *L* = *S*₁ + *L*₀;
when the current position of the inner cutter tube (22) is not at the initial position, the axial movement distance of the inner cutter tube (22) is determined as L = *S*₁ + (*L*₀ *- Lₙ*) when *Lₙ* ≤ *L*₀*,* and the axial movement distance of the inner cutter tube (22) is determined as *L = S*₁ *-* (*Lₙ - L*₀) when *Lₙ* ≥ *L*₀;
where *S*₁ is the set value of the opening length of the sampling window (21a), *L*₀ is a distance from the front end of the inner cutter tube (22) to a front end of the sampling window (21a) when the inner cutter tube (22) is located at the initial position, and *Lₙ* is a distance from the front end of the inner cutter tube (22) to the initial position.

2. The method according to claim 1, wherein the transmission mechanism comprises a transmission member fixedly connected to an output end of the motor (11), the transmission member comprises a first thread segment, the inner cutter tube (22) comprises a second thread segment that threadedly engaged with the first thread segment, and the motor (11) drives the transmission member to rotate, which in turn drives the inner cutter tube (22) to move axially.

3. The method according to claim 1, wherein the transmission mechanism comprises a transmission member fixedly connected to an output end of the motor (11), the transmission member comprises a first thread segment, the transmission mechanism also comprises a transmission sleeve (25) fixedly sleeved on the inner cutter tube (22), the transmission sleeve (25) and the inner cutter tube (22) are arranged to be axially fixed and circumferentially rotate relative to each other, the transmission sleeve (25) is provided with a second thread segment that is threadedly engaged with the first thread segment, and the motor (11) drives the transmission member to rotate, which in turn drives the inner cutter tube (22) to move axially.

4. The method for adjusting the opening size of the sampling window of the biopsy surgical device according to claim 2 or 3, wherein the number X of rotations of the motor corresponding to the axial movement distance L of the inner cutter tube is calculated according to the following method: $X = \frac{L}{P}$ where P is a pitch of the first thread segment or the second thread segment.

5. The method according to claim 1, wherein the transmission mechanism comprises a first driving gear (13) and a first driven gear (23) that mesh with each other, the first driving gear (13) is mounted on an output shaft of the motor (11), the inner cutter tube (22) comprises a third thread segment, the first driven gear (23) comprises a fourth thread segment, the first driven gear (23) and the inner cutter tube (22) are threadedly engaged, and the motor (11) drives the first driving gear (13) to rotate so that the first driven gear (23) drives the inner cutter tube (22) to move axially;

6. The method for adjusting the opening size of the sampling window of the biopsy surgical device according to claim 5, wherein the number X of rotations of the motor (11) corresponding to the axial movement distance L of the inner cutter tube (22) is calculated according to the following method: $X = \frac{L}{{P}_{1} \frac{B}{D}}$ where *P*₁ is a pitch of the third thread segment or the fourth thread segment, B is a number of teeth of the first driving gear (13), and D is a number of teeth of the first driven gear (23).

7. The method according to claim 1, wherein the transmission mechanism comprises a first transmission structure and a second transmission structure, the second transmission structure is configured to drive the inner cutter tube (22) to rotate around an axis thereof, an output portion of the first transmission structure is sleeved on the inner cutter tube (22) and is threaded with the inner cutter tube (22), the output portion and the inner cutter tube (22) rotate in a same direction, the output portion and the inner cutter tube (22) are configured with a speed difference therebetween, and the inner cutter tube (22) is driven to move axially by the speed difference and thread structures on the output portion and the inner cutter tube (22).

8. The method according to claim 7, wherein the first transmission structure comprises a first driving gear (13) and a first driven gear (23) that mesh with each other, the first driving gear (13) is arranged on an output shaft of the motor (11), the inner cutter tube (22) comprises a fifth thread segment, the first driven gear (23) comprises a sixth thread segment, the first driven gear (23) and the inner cutter tube (22) are threadedly engaged, the second transmission structure comprises a second driving gear (14) and a second driven gear (24) that mesh with each other, the second driving gear (14) is arranged on the output shaft of the motor (11), and the second driven gear (24) is sleeved on the inner cutter tube (22) and is arranged to be circumferentially fixed and axially slidable relative to the inner cutter tube (22);

9. The method for adjusting the opening size of the sampling window of the biopsy surgical device according to claim 8, wherein the number X of rotations of the motor (11) corresponding to the axial movement distance L of the inner cutter tube (22) is calculated according to the following method: $X = \frac{L}{{P}_{2} \left(\frac{B}{D}-\frac{A}{C}\right)}$ where *P*₂ is a pitch of the fifth thread segment or the sixth thread segment, B is a number of teeth of the first driving gear (13), D is a number of teeth of the first driven gear (23), A is a number of teeth of the second driving gear (14), and C is a number of teeth of the second driven gear (24).

10. The method according to claim 8, wherein the transmission mechanism further comprises a transmission sleeve (25) fixedly sleeved on the inner cutter tube (22), the transmission sleeve (25) comprises outer threads, and the first driven gear (23) is sleeved on the transmission sleeve (25) and is threadedly engaged with the transmission sleeve (25).

11. The method according to claim 10, wherein an extension segment (26) extends axially from the transmission sleeve (25), one end of the second driven gear (24) is fixedly connected with a sleeve piece (27), the sleeve piece (27) is sleeved on the extension segment (26) and cooperates with the extension segment (26) through a convex-concave structure to transmit torque, and the transmission sleeve (25) is configured to be axially slidably relative to the sleeve piece (27) to maintain torque transmission during a relative axial movement.

12. A control device for adjusting an opening size of a sampling window (21a) of a biopsy surgical device, **characterized in that** the control device comprises:
an obtaining module configured to obtain input instructions, the input instructions at least comprising a set value of an opening length of the sampling window (21a);
a distance calculation module configured to determine an axial movement distance of an inner cutter tube (22) according to a current position of the inner cutter tube (22) and the set value of the opening length of the sampling window (21a);
a rotation number calculation module configured to calculate a number of rotations of a motor (11) corresponding to the axial movement distance of the inner cutter tube (22) according to a transmission relationship of a transmission mechanism; and
a control module configured to control a motor (11) to rotate according to the number of rotations, so that the inner cutter tube (22) moves axially, and the sampling window (21a) reaches a target opening length;
when the current position of the inner cutter tube (22) is an initial position where the front end of the inner cutter tube (22) closes the sampling window (21a), the axial movement distance of the inner cutter tube (22) is determined as L = *S*₁ + *L*₀;
when the current position of the inner cutter tube (22) is not at the initial position, the axial movement distance of the inner cutter tube (22) is determined as L = *S*₁ + (*L*₀ *- Lₙ*) when *Lₙ* ≤ *L*₀*,* and the axial movement distance of the inner cutter tube (22) is determined as *L = S*₁ *-* (*Lₙ - L*₀) when *Lₙ* ≥ *L*₀;
where *S*₁ is the set value of the opening length of the sampling window (21a), *L*₀ is a distance from the front end of the inner cutter tube (22) to a front end of the sampling window (21a) when the inner cutter tube (22) is located at the initial position, and *Lₙ* is a distance from the front end of the inner cutter tube (22) to the initial position.

13. A computer-readable storage medium with a computer program stored therein, wherein when the computer program is executed by a processor, steps of the method according to any one of claims 1 to 11 are implemented.

14. A biopsy surgical device, comprising:
an inner cutter tube (22);
an outer cutter tube (21) defining a sampling window (21a) on a side of a front end thereof, and the outer cutter tube (21) is sleeved over the inner cutter tube (22);
a motor (11) configured to drive the inner cutter tube (22) to move axially through a transmission mechanism and control a front end of the inner cutter tube (22) to stop at any position along an axial direction of the sampling window (21a) of the outer cutter tube (21), thereby adjusting an opening length of the sampling window (21a); and
a controller configured to implement steps of the method for adjusting an opening size of the sampling window (21a) according to any one of claims 1 to 11.

## Patentansprüche

1. Verfahren zum Anpassen einer Öffnungsgröße eines Probenfensters einer chirurgischen Biopsievorrichtung,
wobei die chirurgische Biopsievorrichtung einen Motor (11), ein äußeres Schneidrohr (21), ein inneres Schneidrohr (22) und ein Steuergerät umfasst, wobei das äußere Schneidrohr (21) über das innere Schneidrohr (22) geschoben ist, wobei das Probenfenster (21a) auf einer Seite eines vorderen Endes des äußeren Schneidrohrs (21) definiert ist, wobei der Motor (11) dazu konfiguriert ist, das innere Schneidrohr (22) so anzutreiben, dass es sich axial durch einen Übertragungsmechanismus bewegt, und ein vorderes Ende des inneren Schneidrohrs (22) so zu steuern, dass es an einer beliebigen Position entlang einer axialen Richtung des Probenfensters (21a) des äußeren Schneidrohrs (21) anhält, wodurch eine Öffnungslänge des Probenfensters (21a) angepasst wird;
wobei das Verfahren auf das Steuergerät angewendet wird, **dadurch gekennzeichnet, dass** das Verfahren Folgendes umfasst:
Erhalten einer Eingabeanweisung, die mindestens einen eingestellten Wert einer Öffnungslänge des Probenfensters (21a) umfasst;
Bestimmen einer axialen Bewegungsdistanz des inneren Schneidrohrs (22) gemäß einer aktuellen Position des inneren Schneidrohrs (22) und dem eingestellten Wert der Öffnungslänge des Probenfensters (21a);
Berechnen einer Anzahl von Umdrehungen des Motors (11), die der axialen Bewegungsdistanz des inneren Schneidrohrs (22) entspricht, gemäß einem Übertragungsverhältnis des Übertragungsmechanismus; und
Steuern des Motors (11), um sich gemäß der Anzahl von Umdrehungen zu drehen, wobei bewirkt wird, dass sich das innere Schneidrohr (22) axial bewegt, so dass das Probenfenster (21a) eine Zielfensteröffnungslänge erreicht;
wobei, wenn die aktuelle Position des inneren Schneidrohrs (22) eine Ausgangsposition ist, in der das vordere Ende des inneren Schneidrohrs (22) das Probenfenster (21a) schließt, die axiale Bewegungsdistanz des inneren Schneidrohrs (22) als *L* = *S*₁ + *L*₀ bestimmt wird;
wobei, wenn die aktuelle Position des inneren Schneidrohrs (22) nicht in der Ausgangsposition ist, die axiale Bewegungsdistanz des inneren Schneidrohrs (22) als *L* = *S*₁ + (*L*₀ - *Lₙ)* bestimmt wird, wenn *Lₙ* ≤ *L*₀, und die axiale Bewegungsdistanz des inneren Schneidrohrs (22) als *L = S*₁ *- (Lₙ* - *L*₀) bestimmt wird, wenn *Lₙ ≥ L*₀*;*
wobei *S*₁ der eingestellte Wert der Öffnungslänge des Probenfensters (21a) ist, *L*₀ eine Distanz von dem vorderen Ende des inneren Schneidrohrs (22) zu einem vorderen Ende des Probenfensters (21a) ist, wenn sich das innere Schneidrohr (22) in der Ausgangsposition befindet, und *Lₙ* eine Distanz von dem vorderen Ende des inneren Schneidrohrs (22) zu der Ausgangsposition ist.

2. Verfahren nach Anspruch 1, wobei der Übertragungsmechanismus ein Übertragungselement umfasst, das fest mit einem Ausgangsende des Motors (11) verbunden ist, wobei das Übertragungselement ein erstes Gewindesegment umfasst, wobei das innere Schneidrohr (22) ein zweites Gewindesegment umfasst, das mit dem ersten Gewindesegment in Gewindeeingriff steht, und wobei der Motor (11) das Übertragungselement so antreibt, dass es sich dreht, was wiederum das innere Schneidrohr (22) so antreibt, dass es sich axial bewegt.

3. Verfahren nach Anspruch 1, wobei der Übertragungsmechanismus ein Übertragungselement umfasst, das fest mit einem Ausgangsende des Motors (11) verbunden ist, wobei das Übertragungselement ein erstes Gewindesegment umfasst, wobei der Übertragungsmechanismus auch eine Übertragungshülse (25) umfasst, die fest auf dem inneren Schneidrohr (22) geschoben ist, wobei die Übertragungshülse (25) und das innere Schneidrohr (22) so angeordnet sind, dass sie axial fest sind und sich in Umfangsrichtung relativ zueinander drehen, wobei die Übertragungshülse (25) mit einem zweiten Gewindesegment versehen ist, das mit dem ersten Gewindesegment in Gewindeeingriff steht, und wobei der Motor (11) das Übertragungselement so antreibt, dass es sich dreht, was wiederum das innere Schneidrohr (22) so antreibt, dass es sich axial bewegt.

4. Verfahren zum Anpassen der Öffnungsgröße des Probenfensters der chirurgischen Biopsievorrichtung nach Anspruch 2 oder 3, wobei die Anzahl X von Umdrehungen des Motors, die der axialen Bewegungsdistanz L des inneren Schneidrohrs entspricht, nach dem folgenden Verfahren berechnet wird: $X = \frac{L}{P}$ wobei P eine Steigung des ersten Gewindesegments oder des zweiten Gewindesegments ist.

5. Verfahren nach Anspruch 1, wobei der Übertragungsmechanismus ein erstes Antriebszahnrad (13) und ein erstes angetriebenes Zahnrad (23) umfasst, die miteinander in Eingriff stehen, wobei das erste Antriebszahnrad (13) auf einer Abtriebswelle des Motors (11) montiert ist, wobei das innere Schneidrohr (22) ein drittes Gewindesegment umfasst, wobei das erste angetriebene Zahnrad (23) ein viertes Gewindesegment umfasst, wobei das erste angetriebene Zahnrad (23) und das innere Schneidrohr (22) in Gewindeeingriff stehen, und wobei der Motor (11) das erste Antriebszahnrad (13) so antreibt, dass es sich dreht, so dass das erste angetriebene Zahnrad (23) das innere Schneidrohr (22) so antreibt, dass es sich axial bewegt.

6. Verfahren zum Anpassen der Öffnungsgröße des Probenfensters der chirurgischen Biopsievorrichtung nach Anspruch 5, wobei die Anzahl X von Umdrehungen des Motors (11), die der axialen Bewegungsdistanz L des inneren Schneidrohrs (22) entspricht, nach dem folgenden Verfahren berechnet wird: $X = \frac{L}{{P}_{1} \frac{B}{D}}$ wobei *P*₁ eine Steigung des dritten Gewindesegments oder des vierten Gewindesegments ist, B eine Anzahl von Zähnen des ersten Antriebszahnrads (13) ist und D eine Anzahl von Zähnen des ersten angetriebenen Zahnrads (23) ist.

7. Verfahren nach Anspruch 1, wobei der Übertragungsmechanismus eine erste Übertragungsstruktur und eine zweite Übertragungsstruktur umfasst, wobei die zweite Übertragungsstruktur dazu konfiguriert ist, das innere Schneidrohr (22) so anzutreiben, dass es sich um eine Achse davon dreht, wobei ein Ausgangsabschnitt der ersten Übertragungsstruktur auf dem inneren Schneidrohr (22) geschoben ist und mit dem inneren Schneidrohr (22) verschraubt ist, wobei sich der Ausgangsabschnitt und das innere Schneidrohr (22) in der gleichen Richtung drehen, wobei der Ausgangsabschnitt und das innere Schneidrohr (22) mit einer Geschwindigkeitsdifferenz dazwischen konfiguriert sind, und wobei das innere Schneidrohr (22) so angetrieben wird, dass es sich axial durch die Geschwindigkeitsdifferenz und die Gewindestrukturen auf dem Ausgangsabschnitt und dem inneren Schneidrohr (22) bewegt.

8. Verfahren nach Anspruch 7, wobei die erste Übertragungsstruktur ein erstes Antriebszahnrad (13) und ein erstes angetriebenes Zahnrad (23) umfasst, die miteinander in Eingriff stehen, wobei das erste Antriebszahnrad (13) auf einer Abtriebswelle des Motors (11) angeordnet ist, wobei das innere Schneidrohr (22) ein fünftes Gewindesegment umfasst, wobei das erste angetriebene Zahnrad (23) ein sechstes Gewindesegment umfasst, wobei das erste angetriebene Zahnrad (23) und das innere Schneidrohr (22) in Gewindeeingriff stehen, wobei die zweite Übertragungsstruktur ein zweites Antriebszahnrad (14) und ein zweites angetriebenes Zahnrad (24) umfasst, die miteinander in Eingriff stehen, wobei das zweite Antriebszahnrad (14) auf der Abtriebswelle des Motors (11) angeordnet ist, und wobei das zweite angetriebene Zahnrad (24) auf dem inneren Schneidrohr (22) geschoben ist und so angeordnet ist, dass es in Umfangsrichtung fest ist und relativ zu dem inneren Schneidrohr (22) axial verschiebbar ist.

9. Verfahren zum Anpassen der Öffnungsgröße des Probenfensters der chirurgischen Biopsievorrichtung nach Anspruch 8, wobei die Anzahl X von Umdrehungen des Motors (11), die der axialen Bewegungsdistanz L des inneren Schneidrohrs (22) entspricht, nach dem folgenden Verfahren berechnet wird: $X = \frac{L}{{P}_{2} \left(\frac{B}{D}-\frac{A}{C}\right)}$ wobei *P*₂ eine Steigung des fünften Gewindesegments oder des sechsten Gewindesegments ist, B eine Anzahl von Zähnen des ersten Antriebszahnrads (13) ist, D eine Anzahl von Zähnen des ersten angetriebenen Zahnrads (23) ist, A eine Anzahl von Zähnen des zweiten Antriebszahnrads (14) ist und C eine Anzahl von Zähnen des zweiten angetriebenen Zahnrads (24) ist.

10. Verfahren nach Anspruch 8, wobei der Übertragungsmechanismus ferner eine Übertragungshülse (25) umfasst, die fest auf dem inneren Schneidrohr (22) geschoben ist, wobei die Übertragungshülse (25) Außengewinde umfasst, und wobei das erste angetriebene Zahnrad (23) auf der Übertragungshülse (25) geschoben ist und mit der Übertragungshülse (25) in Gewindeeingriff steht.

11. Verfahren nach Anspruch 10, wobei sich ein Verlängerungssegment (26) axial von der Übertragungshülse (25) erstreckt, wobei ein Ende des zweiten angetriebenen Zahnrads (24) fest mit einem Hülsenstück (27) verbunden ist, wobei das Hülsenstück (27) auf dem Verlängerungssegment (26) geschoben ist und mit dem Verlängerungssegment (26) durch eine konvex-konkave Struktur zusammenwirkt, um Drehmoment zu übertragen, und die Übertragungshülse (25) dazu konfiguriert ist, relativ zu dem Hülsenstück (27) axial verschiebbar zu sein, um die Drehmomentübertragung während einer relativen axialen Bewegung aufrechtzuerhalten.

12. Steuerungsvorrichtung zum Anpassen einer Öffnungsgröße eines Probenfensters (21a) einer chirurgischen Biopsievorrichtung, **dadurch gekennzeichnet, dass** die Steuerungsvorrichtung Folgendes umfasst:
ein Erhaltungsmodul, das dazu konfiguriert ist, Eingabeanweisungen zu erhalten, wobei die Eingabeanweisungen mindestens einen eingestellten Wert einer Öffnungslänge des Probenfensters (21a) umfassen;
ein Distanzberechnungsmodul, das dazu konfiguriert ist, eine axiale Bewegungsdistanz eines inneren Schneidrohrs (22) gemäß einer aktuellen Position des inneren Schneidrohrs (22) und dem eingestellten Wert der Öffnungslänge des Probenfensters (21a) zu bestimmen;
ein Modul zur Berechnung der Anzahl von Umdrehungen, das dazu konfiguriert ist, eine Anzahl von Umdrehungen eines Motors (11), die der axialen Bewegungsdistanz des inneren Schneidrohrs (22) entspricht, gemäß einem Übertragungsverhältnis eines Übertragungsmechanismus zu berechnen; und
ein Steuermodul, das dazu konfiguriert ist, einen Motor (11) so zu steuern, dass er sich gemäß der Anzahl von Umdrehungen dreht, so dass sich das innere Schneidrohr (22) axial bewegt und das Probenfenster (21a) eine Zielöffnungslänge erreicht;
wobei, wenn die aktuelle Position des inneren Schneidrohrs (22) eine Ausgangsposition ist, in der das vordere Ende des inneren Schneidrohrs (22) das Probenfenster (21a) schließt, die axiale Bewegungsdistanz des inneren Schneidrohrs (22) als *L = S*₁ *+ L*₀ bestimmt wird;
wobei, wenn die aktuelle Position des inneren Schneidrohrs (22) nicht in der Ausgangsposition ist, die axiale Bewegungsdistanz des inneren Schneidrohrs (22) als *L* = *S*₁ + (*L*₀ - *Lₙ*) bestimmt wird, wenn *Lₙ* ≤ *L*₀, und die axiale Bewegungsdistanz des inneren Schneidrohrs (22) als *L = S*₁ *- (Lₙ* - *L*₀) bestimmt wird, wenn *Lₙ ≥ L*₀*;*
wobei *S*₁ der eingestellte Wert der Öffnungslänge des Probenfensters (21a) ist, *L*₀ eine Distanz von dem vorderen Ende des inneren Schneidrohrs (22) zu einem vorderen Ende des Probenfensters (21a) ist, wenn sich das innere Schneidrohr (22) in der Ausgangsposition befindet, und *Lₙ* eine Distanz von dem vorderen Ende des inneren Schneidrohrs (22) zu der Ausgangsposition ist.

13. Computerlesbares Speichermedium mit einem darin gespeicherten Computerprogramm, wobei, wenn das Computerprogramm durch einen Prozessor ausgeführt wird, Schritte des Verfahrens nach einem der Ansprüche 1 bis 11 implementiert werden.

14. Chirurgische Biopsievorrichtung, umfassend:
ein inneres Schneidrohr (22);
ein äußeres Schneidrohr (21), das ein Probenfenster (21a) auf einer Seite eines vorderen Endes davon definiert, und wobei das äußere Schneidrohr (21) über das innere Schneidrohr (22) geschoben ist;
einen Motor (11), der dazu konfiguriert ist, das innere Schneidrohr (22) anzutreiben, um sich axial durch einen Übertragungsmechanismus zu bewegen, und ein vorderes Ende des inneren Schneidrohrs (22) so zu steuern, dass es an einer beliebigen Position entlang einer axialen Richtung des Probenfensters (21a) des äußeren Schneidrohrs (21) anhält, wodurch eine Öffnungslänge des Probenfensters (21a) angepasst wird; und
ein Steuergerät, das dazu konfiguriert ist, Schritte des Verfahrens zum Anpassen einer Öffnungsgröße des Probenfensters (21a) nach einem der Ansprüche 1 bis 11 zu implementieren.

## Revendications

1. Procédé permettant l'ajustement d'une taille d'ouverture d'une fenêtre d'échantillonnage d'un dispositif chirurgical de biopsie,
le dispositif chirurgical de biopsie comprenant un moteur (11), un tube de coupe externe (21), un tube de coupe interne (22), et un système de commande, le tube de coupe externe (21) étant manchonné sur le tube de coupe interne (22), la fenêtre d'échantillonnage (21a) étant définie sur un côté d'une extrémité avant du tube de coupe externe (21), le moteur (11) étant configuré pour entraîner le déplacement axial du tube de coupe interne (22) à travers un mécanisme de transmission et commander à une extrémité avant du tube de coupe interne (22) de s'arrêter dans n'importe quelle position le long de la direction axiale de la fenêtre d'échantillonnage (21a) du tube de coupe externe (21), ce qui permet l'ajustement d'une longueur d'ouverture de la fenêtre d'échantillonnage (21a) ;
ledit procédé étant appliqué au système de commande, **caractérisé en ce que** le procédé comprend :
l'obtention d'une instruction d'entrée qui comprend au moins une valeur de consigne d'une longueur d'ouverture de la fenêtre d'échantillonnage (21a) ;
la détermination d'une distance de déplacement axial du tube de coupe interne (22) en fonction de la position courante du tube de coupe interne (22) et de la valeur de consigne de la longueur d'ouverture de la fenêtre d'échantillonnage (21a) ;
le calcul d'un nombre de rotations du moteur (11) correspondant à la distance de déplacement axial du tube de coupe interne (22) conformément à une relation de transmission du mécanisme de transmission ; et
la commande au moteur (11) de tourner en fonction du nombre de rotations, provoquant le déplacement axial du tube de coupe interne (22), de sorte que la fenêtre d'échantillonnage (21a) atteigne une longueur d'ouverture de fenêtre cible ;
lorsque la position courante du tube de coupe interne (22) est une position initiale où l'extrémité avant du tube de coupe interne (22) ferme la fenêtre d'échantillonnage (21a), la distance de déplacement axial du tube de coupe interne (22) est déterminée comme étant *L* = *S*₁ + *L*₀ ;
lorsque la position courante du tube de coupe interne (22) ne se trouve pas dans la position initiale, la distance de déplacement axial du tube de coupe interne (22) est déterminée comme étant *L* = *S*₁ + (*L*₀ - *Lₙ)* lorsque *Lₙ* ≤ *L*₀, et la distance de déplacement axial du tube de coupe interne (22) est déterminée comme étant *L = S*₁ *- (Lₙ* - *L*₀) lorsque *Lₙ* ≥ *L*₀ ;
où *S*₁ représente la valeur de consigne de la longueur d'ouverture de la fenêtre d'échantillonnage (21a), *L*₀ représente une distance entre l'extrémité avant du tube de coupe interne (22) et une extrémité avant de la fenêtre d'échantillonnage (21a) lorsque le tube de coupe interne (22) se trouve dans la position initiale, et *Lₙ* représente une distance entre l'extrémité avant du tube de coupe interne (22) et la position initiale.

2. Procédé selon la revendication 1, ledit mécanisme de transmission comprenant un élément de transmission relié de manière fixe à une extrémité de sortie du moteur (11), ledit élément de transmission comprenant un premier segment de filet, ledit tube de coupe interne (22) comprenant un deuxième segment de filet qui est en prise par filetage avec le premier segment de filet, et ledit moteur (11) entraînant la rotation de l'élément de transmission, ce qui entraîne à son tour le déplacement axial du tube de coupe interne (22).

3. Procédé selon la revendication 1, ledit mécanisme de transmission comprenant un élément de transmission relié de manière fixe à une extrémité de sortie du moteur (11), ledit élément de transmission comprenant un premier segment de filet, ledit mécanisme de transmission comprenant également un manchon de transmission (25) manchonné de manière fixe sur le tube de coupe interne (22), ledit manchon de transmission (25) et ledit tube de coupe interne (22) étant agencés de manière à être axialement fixes et tourner de façon circonférentielle l'un par rapport à l'autre, ledit manchon de transmission (25) étant pourvu d'un deuxième segment de filet qui est en prise par filetage avec le premier segment de filet, et ledit moteur (11) entraînant la rotation de l'élément de transmission, ce qui entraîne à son tour le déplacement axial du tube de coupe interne (22).

4. Procédé permettant l'ajustement de la taille d'ouverture de la fenêtre d'échantillonnage du dispositif chirurgical de biopsie selon la revendication 2 ou 3, ledit nombre X de rotations du moteur correspondant à la distance de déplacement axial L du tube de coupe interne étant calculé conformément au procédé suivant : $X = \frac{L}{P}$ où P représente un pas du premier segment de filet ou du deuxième segment de filet.

5. Procédé selon la revendication 1, ledit mécanisme de transmission comprenant un premier engrenage d'entraînement (13) et un premier engrenage entraîné (23) qui s'engrènent l'un avec l'autre, ledit premier engrenage d'entraînement (13) étant monté sur un arbre de sortie du moteur (11), ledit tube de coupe interne (22) comprenant un troisième segment de filet, ledit premier engrenage entraîné (23) comprenant un quatrième segment de filet, ledit premier engrenage entraîné (23) et ledit tube de coupe interne (22) étant en prise par filetage, et ledit moteur (11) entraînant la rotation du premier engrenage d'entraînement (13) de sorte que ledit premier engrenage entraîné (23) entraîne le déplacement axial du tube de coupe interne (22).

6. Procédé permettant l'ajustement de la taille d'ouverture de la fenêtre d'échantillonnage du dispositif chirurgical de biopsie selon la revendication 5, ledit nombre X de rotations du moteur (11) correspondant à la distance de déplacement axial L du tube de coupe interne (22) étant calculé conformément au procédé suivant : $X = \frac{L}{{P}_{1} \frac{B}{D}}$ où *P*₁ représente un pas du troisième segment de filet ou du quatrième segment de filet, B représente un nombre de dents du premier engrenage d'entraînement (13) et D représente un nombre de dents du premier engrenage entraîné (23).

7. Procédé selon la revendication 1, ledit mécanisme de transmission comprenant une première structure de transmission et une seconde structure de transmission, ladite seconde structure de transmission étant configurée pour entraîner la rotation du tube de coupe interne (22) autour de son axe, une partie de sortie de la première structure de transmission étant manchonnée sur le tube de coupe interne (22) et étant filetée avec le tube de coupe interne (22), ladite partie de sortie et ledit tube de coupe interne (22) tournant dans le même sens, ladite partie de sortie et ledit tube de coupe interne (22) étant configurés avec une différence de vitesse entre eux, et ledit tube de coupe interne (22) étant entraîné pour se déplacer axialement par la différence de vitesse et les structures de filet sur la partie de sortie et le tube de coupe interne (22).

8. Procédé selon la revendication 7, ladite première structure de transmission comprenant un premier engrenage d'entraînement (13) et un premier engrenage entraîné (23) qui s'engrènent l'un avec l'autre, ledit premier engrenage d'entraînement (13) étant agencé sur un arbre de sortie du moteur (11), ledit tube de coupe interne (22) comprenant un cinquième segment de filet, ledit premier engrenage entraîné (23) comprenant un sixième segment de filet, ledit premier engrenage entraîné (23) et ledit tube de coupe interne (22) étant en prise par filetage, ladite seconde structure de transmission comprenant un second engrenage d'entraînement (14) et un second engrenage entraîné (24) qui s'engrènent l'un avec l'autre, ledit second engrenage d'entraînement (14) étant agencé sur l'arbre de sortie du moteur (11), et ledit second engrenage entraîné (24) étant manchonné sur ledit tube de coupe interne (22) et étant agencé de manière à être fixé de façon circonférentielle et axialement coulissant par rapport audit tube de coupe interne (22).

9. Procédé permettant l'ajustement de la taille d'ouverture de la fenêtre d'échantillonnage du dispositif chirurgical de biopsie selon la revendication 8, ledit nombre X de rotations du moteur (11) correspondant à la distance de déplacement axial L du tube de coupe interne (22) étant calculé conformément au procédé suivant : $X = \frac{L}{{P}_{2} \left(\frac{B}{D}-\frac{A}{C}\right)}$ où *P*₂ représente un pas du cinquième segment de filet ou du sixième segment de filet, B représente un nombre de dents du premier engrenage d'entraînement (13), D représente un nombre de dents du premier engrenage entraîné (23), A représente un nombre de dents du second engrenage d'entraînement (14) et C représente un nombre de dents du second engrenage entraîné (24).

10. Procédé selon la revendication 8, ledit mécanisme de transmission comprenant en outre un manchon de transmission (25) manchonné de manière fixe sur le tube de coupe interne (22), ledit manchon de transmission (25) comprenant des filets externes, et ledit premier engrenage entraîné (23) étant manchonné sur le manchon de transmission (25) et étant en prise par filetage avec le manchon de transmission (25).

11. Procédé selon la revendication 10, un segment d'extension (26) s'étendant axialement à partir du manchon de transmission (25), une extrémité du second engrenage entraîné (24) étant reliée de manière fixe à une pièce de manchon (27), ladite pièce de manchon (27) étant manchonnée sur le segment d'extension (26) et coopérant avec le segment d'extension (26) par l'intermédiaire d'une structure convexe-concave de manière à transmettre un couple, et ledit manchon de transmission (25) étant configuré pour être axialement coulissant par rapport à la pièce de manchon (27) de manière à maintenir la transmission de couple pendant un déplacement axial relatif.

12. Dispositif de commande permettant l'ajustement d'une taille d'ouverture d'une fenêtre d'échantillonnage (21a) d'un dispositif chirurgical de biopsie, **caractérisé en ce que** le dispositif de commande comprend :
un module d'obtention configuré pour obtenir des instructions d'entrée, les instructions d'entrée comprenant au moins une valeur de consigne d'une longueur d'ouverture de la fenêtre d'échantillonnage (21a) ;
un module de calcul de distance configuré pour déterminer une distance de déplacement axial d'un tube de coupe interne (22) en fonction de la position courante du tube de coupe interne (22) et de la valeur de consigne de la longueur d'ouverture de la fenêtre d'échantillonnage (21a) ;
un module de calcul du nombre de rotations configuré pour calculer un nombre de rotations d'un moteur (11) correspondant à la distance de déplacement axial du tube de coupe interne (22) conformément à une relation de transmission d'un mécanisme de transmission ; et
un module de commande configuré pour commander à un moteur (11) de tourner en fonction du nombre de rotations, de sorte que le tube de coupe interne (22) se déplace axialement, et que la fenêtre d'échantillonnage (21a) atteigne une longueur d'ouverture cible ;
lorsque la position courante du tube de coupe interne (22) est une position initiale où l'extrémité avant du tube de coupe interne (22) ferme la fenêtre d'échantillonnage (21a), la distance de déplacement axial du tube de coupe interne (22) est déterminée comme étant *L* = *S*₁ + *L*₀ ;
lorsque la position courante du tube de coupe interne (22) ne se trouve pas dans la position initiale, la distance de déplacement axial du tube de coupe interne (22) est déterminée comme étant *L* = *S*₁ + (*L*₀ - *Lₙ)* lorsque *Lₙ* ≤ *L*₀, et la distance de déplacement axial du tube de coupe interne (22) est déterminée comme étant *L = S*₁ *- (Lₙ* - *L*₀) lorsque *Lₙ* ≥ *L*₀ ;
où *S*₁ représente la valeur de consigne de la longueur d'ouverture de la fenêtre d'échantillonnage (21a), *L*₀ représente une distance entre l'extrémité avant du tube de coupe interne (22) et une extrémité avant de la fenêtre d'échantillonnage (21a) lorsque le tube de coupe interne (22) est situé dans la position initiale, et *Lₙ* représente une distance entre l'extrémité avant du tube de coupe interne (22) et la position initiale.

13. Support de stockage lisible par ordinateur dans lequel est stocké un programme informatique, lorsque le programme informatique est exécuté par un processeur, lesdites étapes du procédé selon l'une quelconque des revendications 1 à 11 étant mises en œuvre.

14. Dispositif chirurgical de biopsie, comprenant :
un tube de coupe interne (22) ;
un tube de coupe externe (21) définissant une fenêtre d'échantillonnage (21a) sur un côté de son extrémité avant, et le tube de coupe externe (21) est manchonné sur le tube de coupe interne (22) ;
un moteur (11) configuré pour entraîner le déplacement axial du tube de coupe interne (22) à travers un mécanisme de transmission et commander à une extrémité avant du tube de coupe interne (22) de s'arrêter dans n'importe quelle position le long de la direction axiale de la fenêtre d'échantillonnage (21a) du tube de coupe externe (21), ce qui permet l'ajustement d'une longueur d'ouverture de la fenêtre d'échantillonnage (21a) ; et
un système de commande configuré pour mettre en œuvre les étapes du procédé permettant l'ajustement d'une taille d'ouverture de la fenêtre d'échantillonnage (21a) selon l'une quelconque des revendications 1 à 11.
